(19) Europäisches Patentamt
European Patent Office
Office européen des brevets

(11) Numéro de publication: **0 318 393 A1**

(12) **DEMANDE DE BREVET EUROPEEN**

(21) Numéro de dépôt: **88402968.7**

(51) Int. Cl.[4]: **A 61 K 31/70**

(22) Date de dépôt: **25.11.88**

(30) Priorité: **25.11.87 FR 8716349**

(43) Date de publication de la demande:
**31.05.89 Bulletin 89/22**

(84) Etats contractants désignés:
**AT BE CH DE ES FR GB GR IT LI LU NL SE**

(71) Demandeur: **SANOFI**
**40, Avenue George V**
**F-75008 Paris (FR)**

(72) Inventeur: **Le Fur, Gérard**
**19ter, Rue des Carrières**
**F-95160 Montmorency (FR)**

**Bousquet, Michèle**
**30, Rue des Mathurins**
**F-91570 Bièvres (FR)**

**Crisafulli, Emilio**
**Viale San Gimignano 12**
**I-20146 Milano (IT)**

**Sabadie, Michel**
**Rue de la Fontaine Saint Germain**
**F-27500 Bernay (FR)**

(74) Mandataire: **Gillard, Marie-Louise et al**
**Cabinet Beau de Loménie 55, Rue d'Amsterdam**
**F-75008 Paris (FR)**

(54) **Utilisation de l'adémétionine contre le viellissement de la peau.**

(57) La présente invention a pour objet l'utilisation de l'adémétionine ou d'un de ses sels pour la préparation de compositions pharmaceutiques ou cosmétiques destinées à s'opposer au vieillissement de la peau. Elle concerne également lesdites compositions cosmétiques.



Bundesdruckerei Berlin

## Description

### Utilisation de l'adémétionine contre le vieillissement de la peau.

La présente invention concerne l'utilisation de l'adémétionine et de ses sels pour la préparation de compositions cosmétiques et pharmaceutiques s'opposant au vieillissement de la peau et les compositions cosmétiques ainsi obtenues.

Adémétionine est la Dénomination Commune Internationale du sel interne de l'hydroxyde de (S)-5'-[(3-amino-3-carboxypropyl)-méthylsulfonio]-5'-désoxyadénosine de formule

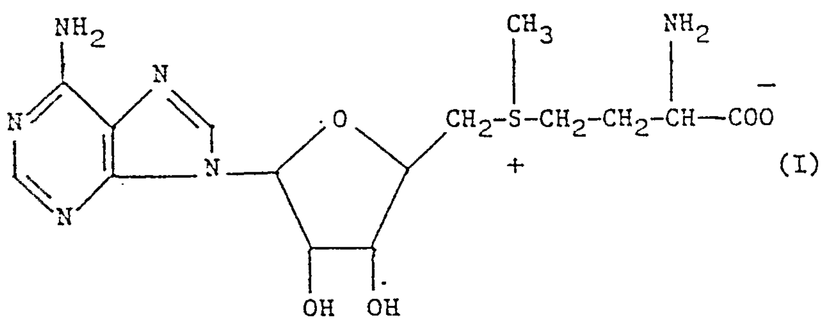

(I)

appelé également S-adénosyl-L-méthionine ou plus simplement SAM.

L'adémétionine est une molécule physiologique de distribution quasi ubiquitaire dans les tissus et dans les liquides de l'organisme où elle intervient dans des processus biologiques importants comme donneur de groupes méthyle dans de nombreuses réactions de transméthylation et comme précurseur de composés soufrés physiologiques tels que le glutathion, la cystéine, la taurine, le CoA.

Il est connu que les taux d'adémétionine sont élevés chez l'enfant et l'adolescent alors qu'ils diminuent chez l'adulte et se réduisent ultérieurement à l'âge présénile et sénile.

L'adémétionine est le principe actif de médicaments notamment pour le traitement des ostéoarthropathies dégénératives où elle joue un rôle important par son activité antiphlogistique et analgésique due à son intervention dans le métabolisme de l'acide arachidonique et des prostaglandines sans interférer avec l'agrégation plaquettaire.

L'adémétionine est également indiquée dans le traitement des syndromes dépressifs.

Ce principe actif n'est ni toxique, ni mutagène et les médicaments le contenant ne présentent pas d'effets secondaires gênants. Ces médicaments sont administrés par voie intramusculaire, intraveineuse ou orale, bien que la possibilité de les administrer comme anti-inflammatoires topiques dans des liquides et pommades dans lesquels le principe actif est dilué dans des solvants ordinaires utilisés en pharmacie ait été prévue (US 4,057,686).

L'adémétionine sous forme de sel interne est instable et est de préférence utilisée, dans les compositions pharmaceutiques, sous forme de sel avec des acides inorganiques et/ou organiques tels que les acides carboxyliques comme le citrate, le tartrate, le malate ou l'ascorbate (FR 2 275 220), les acides minéraux forts ayant un pK inférieur à 2,5 (EP 73 376) et les acides sulfoniques organiques ayant un pK inférieur à 2,5 (EP 72 980, BE 831,310), le sulfate p-toluènesulfonate ou le disulfate di-p-toluènesulfonate étant le sel préféré. Des sels avec des polyanions ont été également proposés (EP 191 133). Pour garantir la bonne stabilité des préparations à base d'adémétionine, il est cependant souhaitable de mélanger le principe actif avec un stabilisant, par exemple, du mannitol (EP 73 376), du lactose ou du maltose (EP 108 817).

On a maintenant trouvé que l'adémétionine a une action s'opposant au vieillissement de la peau.

Plus particulièrement, on a trouvé que l'adémétionine, dissoute ou dispersée dans un véhicule approprié, retarde le vieillissement de la peau en lui donnant également un aspect et une consistance agréables.

Ainsi, selon un de ses aspects, la présente invention concerne l'utilisation de l'adémétionine et de ses sels pour la préparation de compositions pharmaceutiques ou cosmétiques destinées à s'opposer au vieillissement de la peau.

L'action s'opposant au vieillissement est due aux propriétés biochimiques de l'adémétionine. La trans-méthylation se vérifie sur des molécules biologiques, notamment les protéines de la peau, les acides nucléiques et les phospholipides, qui sont biotransformées et entrent dans des cycles anaboliques et cataboliques. Cette activité ana-catabolisante au niveau des cellules épidermiques favorise leur régénération alors que l'action sur les méthyltransferases, par l'augmentation de la méthylation des phospholipides, rend plus fluides les membranes cellulaires ralentissant ainsi le processus naturel de vieillissement de la peau.

Cette action fluidifiant les membranes a été mise en évidence sur des cellules de l'épiderme humain. La microviscosité de membrane des cellules épidermiques humaines a été étudiée selon la méthode de Shnitzkym et al. (J. Biol. Chem. 1974, 249, 2652-2657) en utilisant le diphénylhexatriène comme marqueur fluorescent. La présence d'adémétionine à une concentration molaire de $10^{-6}$ à $10^{-4}$ diminue la viscosité

membranaire de 10 à 25 %.

Selon la présente invention, l'adémétionine peut être utilisée sous forme d'un quelconque des sels indiqués ci-dessus, de préférence le dichlorhydrate, le disulfate, le sel avec 2,5 molécules d'acide sulfurique, le p-toluènesulfonate, le di-p-toluènesulfonate, le tri-p-toluènesulfonate, le disulfate di-p-toluène sulfonate ou les sels avec les polyanions. Parmi ces derniers, les sels avec un sulfate de cellulose tel que le 6-sulfate de cellulose décrit dans EP 116 251, avec un sulfate de chitine tel que le 6-sulfate de chitine décrit dans EP 116 251 ou avec un sulfate de chitosane tel que le 6-sulfate de chitosane décrit dans EP 148 057, sont particulièrement préférés.

Les sels d'adémétionine peuvent être utilisés tels quels ou en présence d'agents stabilisants, par exemple de mannitol, de lactose, de maltose et/ou d'adjuvants comme un acide gras.

Le mannitol intervient généralement dans le rapport : adémétionine ou un de ses sels/mannitol d'environ 3/1.

L'adémétionine et ses sels sont utilisés, pour la préparation des compositions pharmaceutiques ou cosmétiques selon l'invention, dans des quantités suffisant à produire l'effet thérapeutique ou cosmétique, notamment à une concentration de 0,001 à 5 % en poids, de façon souhaitable à une concentration de 0,001 à 1 % et de préférence de 0,05 à 0,2 %.

Le principe actif est mélangé avec des excipients pharmaceutiques ou cosmétiques pour préparer des onguents, des crèmes, des lotions, des émulsions ou des solutions.

Le principe actif peut également être conditionné pour des administrations par voie orale ou parentérale, selon des procédés connus.

Pour préparer des compositions pharmaceutiques à usage local, le principe actif est mélangé aux excipients généralement employés dans la technique pharmaceutique pour les compositions à usage topique tels que, par exemple, les matières grasses d'origine animale, les huiles végétales, les acides gras saturés ou insaturés, les alcools, les glycols polyalkyléniques, les cires, la vaseline, les polyesters qui peuvent être associés à l'eau et àdes agents gélifiants lorsqu'ils sont compatibles. Dans ces préparations, on peut ajouter d'autres ingrédients compatibles avec l'adémétionine et ses sels comme des agents antibactériens ou des parfums.

L'adémétionine, notamment sous forme d'un de ses sels, est de préférence utilisée pour la préparation de compositions cosmétiques destinées à s'opposer au vieillissement de la peau, plus particulièrement destinées à :

- ralentir le vieillissement en maintenant une fluidité optimum des membranes des cellules cutanées, une fluidité membranaire élevée favorisant des écnahges intercellulaires internes donc un métabolisme optimum ;
- améliorer l'état des peaux vieillies prématurément par l'action de facteurs exogènes, selon le processus ci-dessus.

Le sel d'adémétionine est utilisé tel quel ou sous forme de complexe d'encapsulation par exemple du type liposomes, phytosomes, enrobage par une pellicule protectrice ou complexation avec un système protéinique, pour préparer des cosmétiques se présentant sous la forme d'émulsions, de gels aqueux ou anhydres, de systèmes biphasiques à mélange extemporané dans lesquels le sel d'adémétionine est sous forme solide, de produits de maquillage, de lotions à base d'alcool ou non, éventuellement en présence d'un stabilisant.

Selon un autre de ses aspects, la présente invention concerne donc des compositions cosmétiques à base d'adémétionine ou d'un de ses sels. Dans ces compositions, l'adémétionine est de préférence utilisée sous forme de sel, de préférence d'un des sels cités ci-dessus, et, éventuellement, en mélange avec un agent stabilisant tel que le mannitol, le lactose, le maltose ou une cyclodextrine, l'agent stabilisant préféré étant le mannitol.

Lorsqu'on utilise un mélange sel d'adémétionine / stabilisant, le mélange préféré est adémétionine disulfate di-p-toluènesulfonate/mannitol dans un rapport 2:1 à 4:1, de préférence 3:1.

Les compositions cosmétiques de la présente invention peuvent également contenir des adjuvants tels que des acides gras, saturés ou insaturés, non substitués ou substitués avec un groupe hydroxyle, contenant de 1 à 18 atomes de carbone, ou un sel alcalin desdits acides gras.

Dans ces compositions, la concentration peut être de 0,001 à 5 % ; la concentration souhaitable est de 0,001 à 1 %, de préférence de 0.05 à 0,2 % en poids.

La forme des compositions cosmétiques selon l'invention peut être une crème dans laquelle l'adémétionine, un de ses sels ou un mélange d'un de ces produits avec le stabilisant est associé aux excipients couramment utilisés dans la cosmétologie, et compatibles avec l'adémétionin et ses sels, tels que la lanoline.

Les compositions cosmétiques de l'invention peuvent être également présentées sous forme de gel dans les excipients appropriés tels que les esters de cellulose, les esters d'acides gras, par exemple le myristate d'isopropyle ou d'autres agents gélifiants.

Les compositions cosmétiques suivant l'invention peuvent aussi prendre la forme de lotion ou solution dans laquelle l'adémétionine, son sel ou le mélange d'un de ces produits avec le stabilisant est dissout ou microdispersé dans une microémulsion.

La forme des compositions pharmaceutiques ou cosmétiques suivant l'invention peut donc être une microdispersion d'adémétionine dans un liquide contenant de l'eau, ainsi qu'un ou plusieurs agents tensioactifs. Ces dispersions présentent les caractères des microémulsions et ont pratiquement l'aspect de solutions vraies. Elles sont de préférence préparées extemporanément.

Ces microémulsions jouissent d'une bonne stabilité et peuvent être conservées pendant le temps

nécessaire pour l'utilisation à des températures comprises entre 0 et 60° C sans qu'il y ait sédimentation des constituants ou séparation de phases.

Les tensioactifs de la composition sont choisis parmi les agents de surface utilisables en cosmétologie. On peut indiquer à titre d'exemples non limitatifs : esters de sorbitol et leurs dérivés polyoxyéthylénés, les huiles de ricin (hydrogénées ou non) polyoxyéthylénées, les copolymères séquencés oxyde d'éthylène/oxyde de propylène, les alcools gras polyoxyéthylénés, le laurylsulfate de sodium, le dioctylsulfosuccinate de sodium, les lécithines d'oeuf ou de soja.

Lorsque l'adémétionine est utilisée sous forme de sel, si l'on souhaite maintenir le pH voisin de la neutralité, on peut ajouter à la composition un agent de neutralisation. L'agent neutralisant éventuel peut être constitué soit par un mélange tampon par exemple un tampon phosphate, soit simplement par une amine biocompatible telle que la mono-, la di- ou la tri-éthanolamine.

Selon un mode de réalisation préférée, en utilisant des agents tensioactifs dont la Balance Hydrophile Lipophile (HLB) est comprise entre 10 et 17 et de préférence entre 11 et 15, dans un pourcentage de 7 à 40 %, on obtient des microémulsions diluables dans l'eau en toutes proportions et ce, quel que soit le degré hydrométrique de l'eau utilisée.

De telles dilutions restent stables pendant plusieurs jours, ce qui est très suffisant compte tenu de leur utilisation.

Les compositions pharmaceutiques ou cosmétiques de la présente invention sont très bien tolérées, elles ne présentent aucune phototoxicité et leur application sur la peau pour des périodes de temps prolongées n'implique aucun effet systémique. En général, après trente jours d'application, on note une amélioration de l'aspect de la peau et un ralentissement dans la formation des rides.

Les exemples suivants illustrent l'invention sans toutefois la limiter. Dans ces exemples, on utilisera les abréviations suivantes :

| | |
|---|---|
| SAM = | adémétionine, |
| SAM-2s,2t = | adémétionine disulfate, di-p-toluènesulfonate, |
| SAM-2s = | adémétionine disulfate. |

Dans un souci de simplification, certains constituants des compositions ont été désignés par leur dénomination commerciale dont voici les significations :

| | |
|---|---|
| Aerosil 200 : | aérosol de silice, commercialisé par DEGUSSA |
| Solutol HS 15 : | 12-hydroxystéarate de polyéthylèneglycol 600, commercialisé par BASF |
| Labrafil 1944 CS : | triglycérides d'acides gras insaturés polyoxyéthylénés, commercialisé par GATTEFOSSE |
| Acrysol : | copolymère acylates/méthacrylates, commercialisé par SEPPIC |
| Finsolv TN : | benzoates d'alcools contenant 12-15 . atomes de carbone, commercialisé par WITCO |
| Cetiol HE : | polyéthylèneglycol-7 glycéryl cocoate, commercialisé par HENKEL |
| Labrafac hydrophile : | triglycérides contenant 7-8 atomes de carbone polyoxyéthylénés, commercialisé par GATTEFOSSE |
| Abil 8551 B : | diméthicone copolyol, commercialisé par GOLDSCHMIDT |
| Carbopol 934 : | carboxypolyméthylène, commercialisé par GOLDSCHMIDT |
| Carbopol 940 : | carboxypolyméthylène, commercialisé par GOLDSCHMIDT |
| EDTA : | acide éthylènediaminotétracétique |
| Filtre UVA-UVB : | 4-méthoxycinnamate d'éthyle-2 hexyle (marque PARFOL MCX). |

EXEMPLE 1

Gel à base d'un mélange de SAM disulfate, ditosilate/mannitol 3/1, à 1 % de SAM base

| | |
|---|---|
| SAM-2s,2t | 2,88 g |
| Aerosil 200 | 7,00 g |
| Myristate d'isopropyle à | 100,00 g |

EXEMPLE 2

Microémulsion pour préparation extemporanée

| | |
|---|---|
| SAM-2s,2t | 0,1 % |
| Solutol HS 15 | 2,0 % |
| Labrafil 1944 CS | 1,0 % |
| Eau q.s.p. | 100,0 % |

La microémulsion est préparée en mélangeant l'ensemble des excipients et agitant jusqu'à l'obtention d'une solution limpide.

Le composant SAM-2s,2t est ajouté au moment de l'utilisation et la microémulsion ainsi obtenue reste stable plusieurs jours.

EXEMPLE 3

Microémulsion pour préparation extemporanée obtenue comme décrit dans l'exemple 2.

| | |
|---|---|
| SAM-2s,2t | 0,1 % |
| Solutol HS 15 | 4,0 % |
| Labrafil 1944 CS | 2,0 % |
| Eau q.s.p. | 100,0 % |

EXEMPLE 4

Microémulsion pour préparation extemporanée obtenue comme décrit dans l'exemple 2.

| | |
|---|---|
| SAM-2s,2t | 0,1 % |
| Finsolv TN | 5,5 % |
| Solutol HS 15 | 8,0 % |
| Eau q.s.p. | 100,0 % |

EXEMPLE 5

Microémulsion pour préparation extemporanée obtenue comme décrit dans l'exemple 2.

| | |
|---|---|
| SAM-2s,2t | 0,1 % |
| Solutol HS 15 | 4,0 % |
| Labrafil 1944 CS | 2,0 % |
| Hydroxyéthyl cellulose | 0,5 % |
| Eau q.s.p. | 100,0 % |

EXEMPLE 6

Microémulsion pour préparation extemporanée obtenue comme décrit dans l'exemple 2.

| | |
|---|---|
| SAM-2s,2t | 0,1 % |
| Solutol HS 15 | 4,0 % |
| Labrafil 1944 CS | 2,0 % |
| Hydroxyéthyl cellulose | 1,0 % |
| Eau q.s.p. | 100,0 % |

EXEMPLE 7

Microémulsion pour préparation extemporanée obtenue comme décrit dans l'exemple 2.

| | |
|---|---|
| SAM-2s,2t | 0,1 % |
| Solutol HS 15 | 4,0 % |
| Labrafil 1944 CS | 2,0 % |
| Acrysol | 1,0 % |
| Eau q.s.p. | 100,0 % |

EXEMPLE 8

Microémulsion pour préparation extemporanée obtenue comme décrit dans l'exemple 2.

| | |
|---|---|
| SAM-2s,2t | 0,1 % |
| Solutol HS 15 | 4,0 % |
| Labrafil 1944 CS | 2,0 % |
| Acrysol | 2,0 % |
| Eau q.s.p. | 100,0 % |

EXEMPLE 9

Microémulsion pour préparation extemporanée obtenue comme décrit dans l'exemple 2.

| | |
|---|---|
| SAM-2s,2t | 0,10 % |
| Solutol HS 15 | 1,00 % |
| Labrafac Hydrophile | 0,25 % |
| Cetiol HE | 0,20 % |
| Abil 8551 B | 0,05 % |
| Propylèneglycol | 12,50 % |
| Ethanol | 12,50 % |
| Carbopol 934 | 0,40 % |
| Triéthanolamine q.s.p. | pH 6 |
| Parfum q.s. | |
| Colorant q.s. | |
| Eau q.s.p. | 100,0 % |

EXEMPLE 10

Préparation extemporanée

A/ Solvant:

| | |
|---|---|
| Carboxyméthylcellulose | 0,30 g |
| Conservateurs dans propylène glycol : | 5,00 g |
| Phénoxyéthanol 0,5 g | |
| 4-hydroxybenzoate de méthyle 0,1 g | |
| 4-hydroxybenzoate d'éthyle 0,1 g | |
| 4-hydroxybenzoate de propyle 0,1 g | |
| 4-hydroxybenzoate de butyle 0,1 g | |
| Propylène glycol 4,1 g | |
| Huile de ricin hydrogénée éthoxylée | 1,00 g |
| Parfum | 0,20 g |
| Eau déminéralisée q.s.p. | 100,00 g |

B/ Poudre :

| | |
|---|---|
| SAM-2s | 0,01 g |
| Lactose q.s.p. | 100,00 g |

EXEMPLE 11

Crème de nuit

| | |
|---|---|
| Alcool cétylique | 2,00 g |
| Stéarine | 2,50 g |
| Monostéarate de glycérol | 5,00 g |
| Palmitate d'isopropyle | 5,00 g |
| Huile végétale | 3,00 g |
| Huile minérale | 2,00 g |
| Perhydrosqualène | 2,00 g |
| Huile de solicone | 1,00 g |
| Beurre de karité | 2,00 g |
| Palmitate d'éthyle-2 hexyle | 5,00 g |
| Triéthanolamine | 5,50 g |
| Conservateurs dans butylène glycol : | 5,00 g |
| Phénoxyéthanol 0,5 g | |
| 4-hydroxybenzoate de méthyle 0,1 g | |
| 4-hydroxybenzoate d'éthyle 0,1 g | |
| 4-hydroxybenzoate de propyle 0,1 g | |
| 4-hydroxybenzoate de butyle 0,1 g | |
| Butylène glycol 4,1 g | |
| EDTA tétrasodique | 0,10 g |
| Parfum | 0,30 g |
| SAM-2s | 0,01 g |
| Eau q.s.p. | 100,00 g |

EXEMPLE 12

Crème de jour protectrice

| | |
|---|---|
| Monostéarate de sorbitan éthoxylé | 2,60 g |
| Huile de silicone | 1,00 g |
| Alcool cétylique | 2,00 g |
| Huile minérale | 3,00 g |
| Alcool de lanoline | 1,00 g |
| Perhydrosqualène | 1,00 g |
| Monostéarate de sorbitan | 2,40 g |
| Palmitate de cétyle | 3,00 g |
| Palmitate d'isopropyle | 4,00 g |
| Filtre UVA-UVB | 2,00 g |
| EDTA tétrasodique | 0,10 g |
| Carbopol | 0,30 g |
| Triéthanolamine | 0,30 g |
| Alpha-tocophérol | 0,01 g |
| Conservateurs dans butylène glycol : | 5,00 g |
| Phénoxyéthanol 0,5 g | |
| 4-hydroxybenzoate de méthyle 0,1 g | |
| 4-hydroxybenzoate d'éthyle 0,1 g | |
| 4-hydroxybenzoate de propyle 0,1 g | |
| 4-hydroxybenzoate de butyle 0,1 g | |
| Butylène glycol 4,1 g | |
| Parfum | 0,30 g |
| SAM-2s | 0,01 g |
| Eau déminéralisée q.s.p. | 100,00 g |

EXEMPLE 13
Gel

| | |
|---|---|
| Carbopol 940 | 0,20 g |
| Polyéthylène glycol | 3,00 g |
| Alpha-tocophérol | 0,01 g |
| Conservateurs dans butylène glycol : | 5,00 g |
| Phénoxyéthanol 0,5 g | |
| 4-hydroxybenzoate de méthyle 0,1 g | |
| 4-hydroxybenzoate d'éthyle 0,1 g | |
| 4-hydroxybenzoate de propyle 0,1 g | |
| 4-hydroxybenzoate de butyle 0,1 g | |
| Butylène glycol 4,1 g | |
| Parfum | 0,30 g |
| Triéthanolamine | 0,25 g |
| SAM-2s | 0,01 g |
| Eau déminéralisée q.s.p. | 100,00 |

EXEMPLE 14
Base fluide de maquillage

| | |
|---|---|
| Stérols de soja éthoxylés | 4,00 g |
| Stérols de soja | 0,50 g |
| Monostéarate de glycérol | 1,00 g |
| Huile végétale | 1,50 g |
| Palmitate d'éthyle-2 hexyle | 4,00 g |
| Alcool céthylique | 0,50 g |
| Triglycérides capriques/capryliques | 1,50 g |
| Huile de silicone | 1,00 g |
| Huile minérale | 1,80 g |
| Alcool de lanoline | 0,20 g |
| Dipélargonate de propylène glycol | 3,00 g |
| Lécithine | 1,00 g |
| Conservateurs dans butylène glycol : | 5,00 g |
| Phénoxyéthanolamine 0,5 g | |
| 4-hydroxybenzoate de méthyle 0,1 g | |
| 4-hydroxybenzoate d'éthyle 0,1 g | |
| 4-hydroxybenzoate de propyle 0,1 g | |
| 4-hydroxybenzoate de butyle 0,1 g | |
| Butylène glycol 4,1 g | |
| Carbopol | 0,20 g |
| Triéthanolamine | 0,20 g |
| EDTA tétrasodique | 0,10 g |
| Alpha-tocophérol | 0,01 g |
| Parfum | 0,30 g |
| SAM-2s | 0,01 g |
| Eau déminéralisée q.s.p. | 100,00 g |

## Revendications

1. Utilisation de l'adémétionine ou d'un de ses sels pour la préparation de compositions pharmaceutiques ou cosmétiques destinées à s'opposer au vieillissement de la peau.

2. Utilisation selon la revendication 1, caractérisée en ce que le sel d'adémétionine est le disulfate di-p-toluènesulfonate ou le disulfate.

3. Utilisation selon la revendication 1, caractérisée en ce que le sel d'adémétionine est un sel avec un polyanion.

4. Utilisation selon la revendication 3, caractérisée en ce que le polyanion est un sulfate de cellulose, un sulfate de chitine ou le chitosane 6-sulfate.

5. Utilisation selon l'une quelconque des revendications 1 à 4, caractérisée en ce que l'adémétionine ou son sel est mélangée à un agent stabilisant tel que le mannitol, le lactose ou le maltose.

6. Utilisation selon la revendication 5, caractérisée en ce que le rapport adémétionine, ou sel d'adémétionine/mannitol est environ 3/1.

7. Utilisation selon l'une quelconque des revendications 1 à 6 pour la préparation extemporanée de compositions pharmaceutiques ou cosmétiques destinées à s'opposer au vieillissement de la peau, sous forme de microémulsions.

8. Utilisation selon l'une quelconque des revendications précédentes, pour la préparation de compositions contenant de 0,001 à 5 % en poids, généralement de 0,001 à 1 % en poids et de préférence de 0,05 à 0,2 % en poids d'adémétionine ou d'un de ses sels.

9. Composition cosmétique caractérisée en ce qu'elle renferme de l'adémétionine ou l'un de ses sels.

10. Composition cosmétique selon la revendication 9, caractérisée en ce qu'elle renferme en outre un

agent stabilisant.

11. Composition cosmétique selon la revendication 10, caractérisée en ce qu'elle renferme un mélange adémétionine disulfate di-p-toluènesulfonate/mannitol dans un rapport 3/1 environ.

12. Composition selon l'une des revendications 9 ou 10, caractérisée en ce qu'elle renferme l'adémétionine disulfate.

13. Composition selon l'une quelconque des revendications 9 à 12, caractérisée en ce qu'elle contient l'adémétionine ou son sel à une concentration de 0,001 à 5 % en poids dans un excipient cosmétique.

14. Composition selon la revendication 13, caractérisée en ce qu'elle contient l'adémétionine ou son sel à une concentration de 0,001 à 1 % en poids, de préférence 0,05 à 0,2 % en poids.

Office européen des brevets

# RAPPORT DE RECHERCHE EUROPEENNE

Numero de la demande

EP 88 40 2968

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| Y | DIALOG MEDLINE, no. 80119134; F.K. LIN et al.: "Differences between transfer RNA methylase activity in human diploid fibroblasts during in vitro and in vivo aging", & MECH. AGEING DEV., décembre 1979, 11(5-6), 383-92<br>* Résumé *<br>--- | 1 | A 61 K 31/70 |
| Y | DIALOG MEDLINE, no. 77124804; B. BUEHLER et al.: "Ornithine decarboxylase and S-adenosyl methionine decarboxylase in skin fibroblasts of normal and cystic fibrosis patients", & PEDIATR. RES., mar 1977, 11(3 Pt 1), 186-90<br>* Résumé *<br>--- | 1 | |
| D,Y | BE-A- 831 310 (ERREKAPPA)<br>* Revendications; page 32, lignes 14-40; page 33, lignes 1-39 *<br>----- | 1 | |
| | | | DOMAINES TECHNIQUES RECHERCHES (Int. Cl.4) |
| | | | A 61 K 31/00 |

Le présent rapport a été établi pour toutes les revendications

| Lieu de la recherche | Date d'achèvement de la recherche | Examinateur |
|---|---|---|
| LA HAYE | 20-02-1989 | SCARPONI U. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)